# EUROPEAN PATENT SPECIFICATION

(11) **EP 4 580 564 B1**
(45) Date of publication and mention of the grant of the patent: **08.04.2026**
(21) Application number: 23767949.3
(22) Date of filing: 30.08.2023
(51) Int. Cl.: A61F 6/14

(54) **INTRA-UTERINE MONITORING AND CONTRACEPTIVE SYSTEM**
INTRAUTERINES ÜBERWACHUNGS- UND EMPFÄNGNISVERHÜTUNGSSYSTEM
SYSTÈME DE SURVEILLANCE INTRA-UTÉRINE ET DE CONTRACEPTION

(30) Priority: 31.08.2022 GB 202212640
(43) Date of publication of application: 09.07.2025
(73) Proprietor: Verso Biosense Limited, Milton Abingdon, Oxfordshire OX14 4RZ (GB)
(72) Inventor: CEFAI, Joseph, Swansea West Glamorgan SA2 8AX (GB); DICKSON, Charles, Abingdon Oxfordshire OX14 4RZ (GB)
(74) Representative: Worthington, Richard Easton
(86) International application number: PCT/GB2023/052237
(87) International publication number: WO 2024/047342

(56) References cited:
- WO-A1-2018/162868
- WO-A1-2022/029780
- WO-A2-2019/122937
- US-A1- 2017 340 476

## Description

### Technical Field

The present invention relates to an intra-uterine monitoring and contraceptive system. Embodiments of the present invention relate to a system for both long-term, real-time, *in-vivo* measurement of biophysical parameters in a human uterus and contraceptive functions.

### Background

Contraceptive intra-uterine devices (IUDs) are implantable contraceptive devices. Currently, contraceptive IUDs either chemically or physically disrupt the uterus to prevent implantation of a fertilised egg. They are effective contraceptive devices as they do not rely on the patient performing a task (e.g. taking an oral contraceptive pill daily), are long term (>5 years), and are non-permanent. Contraceptive IUDs are designed to be implanted in a uterus for months to years, and during this time, uterine function and the intra-uterine environment (i.e. biophysical parameters, such as temperature, dissolved oxygen concentration, pH, glucose, etc) of the uterus may change, e.g. in response to hormonal changes as the patient ages. Abnormal changes in the intra-uterine environment may act as early-stage indicators for disease, such as endometrial carcinoma, or conditions such as endometriosis. However, current contraceptive IUDs cannot provide long-term in vivo monitoring to detect these changes in the uterus.

The present invention is intended to address these limitations.

WO2018/162868 A1 describes an intra-uterine monitoring system with an implantable sensor device, shaped and dimensioned for implantation in a uterus for measuring conditions within the uterus to generate sensor data, and a wearable receiver device, for wirelessly receiving the sensor data generated by the implantable sensor device.

### Summary of the Invention

According to an aspect of the present invention, there is provided an intra-uterine system according to claim 1.

Different embodiments are illustrated in the various following examples.

The IUD can monitor and collect data of the intra-uterine environment for extended periods of time. The intra-uterine device (IUD) can be kept small and simple, requiring only the mechanical and electronic structures necessary to take sensor measurements and transmit those to the receiver device, and means to either chemically or physically disrupt the intra-uterine environment to prevent implantation of a fertilised egg.

Preferably, the receiver device is operable to wirelessly charge the IUD. In this way, a battery may not be required in the IUD, or its size may be minimised, enabling it to be made smaller, and avoiding potential problems associated with battery leakage within the uterus.

Preferably, the IUD is shaped so as to anchor in the uterus. This may prevent movement of the IUD within the uterus to ensure effective contraceptive function, and may assist with effective wireless communication with the receiver device. Preferably, the IUD is shaped to contact at least part of the uterine lining in the uterus.

Preferably, the at least part of the IUD which contacts the uterine lining disrupts the growth of the uterine lining so as to affect implantation of a fertilised egg to prevent pregnancy.

Preferably, the IUD releases a chemical into the uterus to disrupt the intra-uterine environment.

Preferably, the chemical is provided in or as a coating of a part of the intra-uterine device, or in or as a part of the intra-uterine device.

Preferably, the chemical is hormonal, e.g. progestin or estrogen, or nonhormonal, e.g. copper.

Preferably, the wireless charging may be conducted using electromagnetic field coupling wireless energy transfer. The receiver device may comprise an antenna, transceiver circuitry and a power source, and the IUD may comprise an antenna, a charging circuit and a controller. The receiver device may be operable to transmit electrical power from the antenna of the receiver device to the antenna of the IUD via electromagnetic coupling. The electrical power is used by the charging circuit to store electrical power for operating the sensors of the IUD and for transmitting sensor data to the receiver device.

Preferably, the IUD comprises a capacitor or a rechargeable battery, and the charging circuit may store the electrical power by charging the capacitor or the rechargeable battery.

The power source may be a rechargeable battery.

Preferably, the receiver device has a controller, and a primary coil for wirelessly communicating with the intra-uterine device, the receiver device being operable to wirelessly charge the intra-uterine device via inductive coupling between the primary and secondary coils; wherein:
a quality factor of the primary coil is controllable;
the controller is operable to control the quality factor of the primary coil to be higher when the receiver device is wirelessly charging the intra-uterine device than when the receiver device is receiving sensor data from the intra-uterine device; and the receiver device drives the primary coil to operate at a frequency of order 100kHz.

As a result of the quality factor being controllable, the same coil can be used both for efficient power transfer (wireless charging) by using the coil in a (relatively) high quality factor mode, and for reliable data communications by using the coil in a (relatively) low quality factor mode.

The quality factor may be controllable by modifying an electrical resistance associated with the primary coil. For example, a damping resistor could be switched in and out of series with the primary coil.

The quality factor of the primary coil may be selectable between a plurality of different levels. In a simple case, this could be two levels - a high level for wireless charging and a low level for receiving and/or transmitting data. In a more complicated case, this could be three levels, with an intermediate quality factor being used, for example if a different quality factor would be optimal for transmitting data compared with receiving data. For example, different data rates may be used for transmitting and receiving. If a faster data rate is used for receiving, then receiving will require a higher bandwidth and thus a lower quality factor. All other factors being equal, a coil with relatively high quality factor will output a higher field strength than a relatively low quality factor for the same input power. This will make the wireless charging more efficient. A coil with a relatively high quality factor corresponds to a lower resistance in the coil circuit, which will result in less heat generated than a low quality factor coil at the same input power. This has benefits for heat and thermal distribution within the receiver device.

Another reason for providing multiple different quality factors is to provide robustness to the communication link when the user is close to a metallic environment. For example, if the user is sitting in a car or sitting in a metal chair, then if the system is operating at a high quality factor, the presence of metal may detune the system, causing one or both of power transfer and communication to fail. However, a system with a lower quality factor will waste more power, which is generally undesirable, and may be unacceptable for a device, where power consumption needs to be relatively low. In order to alleviate this problem, the system may be configured to adaptively change the quality factor to accommodate the external environment while maintaining a reasonable battery life.

The receiver device may comprise a coil circuit, the coil circuit comprising the primary coil and a damping resistor, the damping resistor being switched into series with the primary coil to reduce the quality factor of the primary coil. The coil circuit may comprise one or more further damping resistors, different ones or combinations of the damping resistors being used to control an amount of reduction of the quality factor.

Preferably, the IUD comprises a single axis antenna, and wherein the receiver device comprises Helmholtz coils, wherein the Helmholtz coils are arranged to communicate with the single axis antenna so as to wirelessly charge the intra-uterine device and receive the sensor data.

The single axis antenna may be configured to operate at a frequency of the order of 100kHz, preferably in the range 119kHz to 140kHz, preferably 130kHz to 140kHz, preferably 139.6kHz.

The IUD may comprise one or more of a temperature sensor, a pH sensor and a dissolved oxygen sensor. These sensors, which may be miniaturised electrochemical sensors integrated into the IUD, are particularly appropriate for human fertility analysis applications. Other biophysical parameter sensors could also be used, for example electrical conductivity or pressure sensors could be provided. In some cases, multiple sensors of the same type (for example temperature sensors) may be provided at different locations on the IUD, for example in the form of a sensor array. This enables separate measurements to be made at different regions within the uterus. For example, a first temperature sensor could be provided at one end of the IUD, proximate the cervix, while a second temperature sensor could be provided at the other end of the IUD, further into the uterus. In this way, a temperature distribution, or gradient, can be inferred.

The IUD may comprise a body and one or more arms, the arms projecting laterally from the body to secure the sensor within the uterus. This should increase the likelihood of the device remaining in place throughout the desired implantation period. The IUD may comprise a pair of arms positioned at or proximate one end of the body and extending generally away from each other. These structures have been found to provide stable positioning of the IUD within a uterus.

The receiver device may be a wearable receiver device.

By making the receiver device wearable, it can be kept in relatively close proximity to the IUD on a long-term basis, making regular monitoring easier.

The receiver device may be provided in a garment. Garments can be an effective means of ensuring the receiver device is held adjacent the user in a generally fixed position for long periods.

The garment may be underwear, e.g. worn adjacent the uterus, or may be a belt to be worn around the waist of a user. Alternatively, the wearable receiver device may be a sanitary pad. As a result, the system provides negligible interference to a users' daily life. The garment is also located in relatively close proximity to the IUD. The receiver device may be a mat or blanket. Such a device may allow monitoring and/or charging for long periods, for example whilst the user is lying down or sleeping.

The wearable receiver device may comprise a transmitter for wirelessly transmitting received sensor data to an external device. The external device may be a portable electronic device (such as a mobile telephone, a tablet, or a dedicated handset) or a computer. The external device may be a remote server, or a database for building up a library of sensor data.

The intra-uterine device has, according to the invention, antenna arranged internally within the intra-uterine device.

The intra-uterine device has, according to the invention, a contraceptive copper portion provided externally on the intra-uterine device.

The contraceptive copper portion may be spatially separated from the antenna such that the contraceptive copper portion does not impede the antenna operation.

### Brief Description of the Drawings

Embodiments of the present invention will now be described by way of example only with reference to the accompanying drawings where like parts are provided with corresponding reference numerals and in which:
Figure 1 schematically illustrates an intra-uterine system according to an embodiment of the invention;
Figure 2 schematically illustrates an IUD according to one embodiment;
Figure 3 schematically illustrates an IUD according to another embodiment;
Figure 4A schematically illustrates a front view of a wearable garment according to one embodiment;
Figure 4B schematically illustrates a back view of the wearable garment according to the embodiment of Figure 4A;
Figure 5 schematically illustrates the wearable garment of the embodiment of Figure 4A in use;
Figure 6 schematically illustrates an inductive coupling between a sensor device and a receiver device;
Figure 7 is a schematic block diagram of an implantable sensor device; and
Figure 8 is a more detailed schematic block diagram of an implantable sensor device.

### Detailed Description

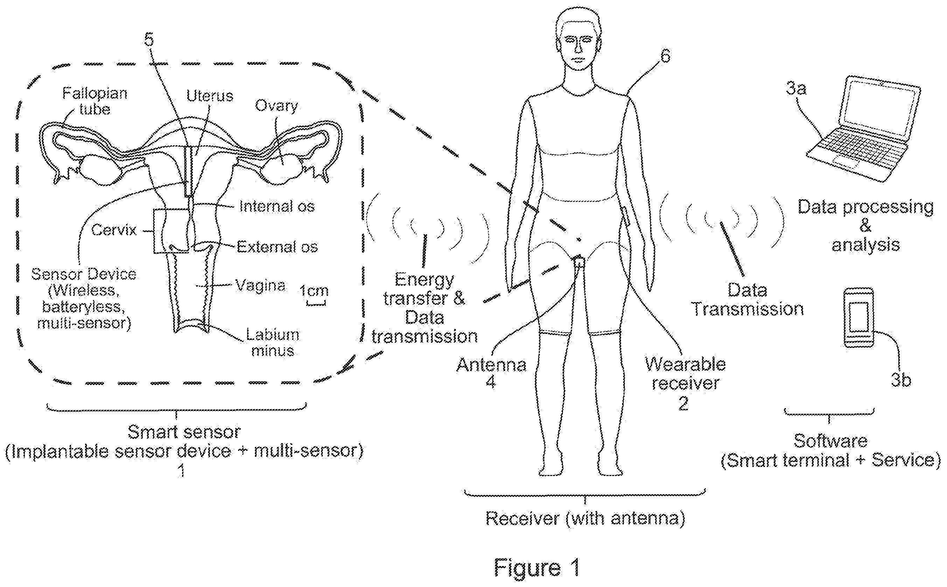

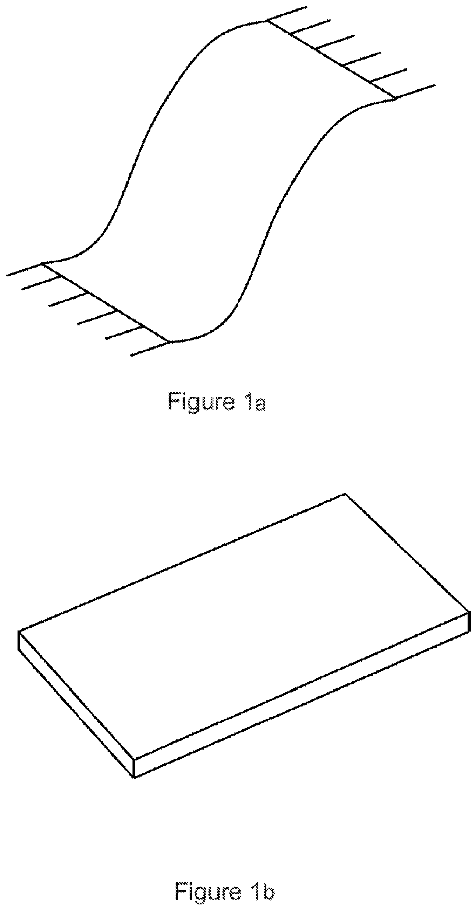

Referring to Figure 1, a three-module structured multi-parameter *in-vivo* sensing platform for intra-uterine environment monitoring is shown. The platform comprises a smart IUD sensor 1, which is suitably shaped and dimensioned for implantation in a human uterus, an external receiver 2 and monitoring software installed on suitable data processing hardware, such as a computer 3a or portable electronic device 3b.

Whilst in the present example shown in Figure 1, the receiver device 2 is a wearable receive device 2 provided in a garment, the receiver device 2 may take any suitable form. The receiver device 2 may be a mat (for example as shown in Figure 1a) or blanket (for example as shown in Figure 1b), or any other suitable means that allows prolonged communication between the IUD sensor 1 and the receiver device 2.

The smart IUD sensor 1 is a fully implantable (within the uterus 5 of a human female body 6) sensor device incorporating multiple embedded biosensors (intended for measuring hormones, proteins, ions, glucose, lactate, temperature, dissolved oxygen concentration (DOC) and pH). These parameters may be monitored over a period of time to act as a diagnosis tool. For example, depletion of oxygen (determined by the dissolved oxygen concentration) may be indicative of endometrial carcinomas. This can be detected at a much earlier rate as these carcinomas are often not linked to pain. Similarly, excess estradiol may be indicative of endometritis.

The smart IUD sensor 1 acts as a contraceptive device. The sensor 1 does so by either chemical disruption, physical disruption, or a combined disruptive mechanism. For chemical disruption, the sensor 1 releases a hormone into the uterus to disrupt the intra-uterine environment. In one example, the hormone is progestin. This acts as contraceptive to prevent pregnancy in two ways: firstly, the excess progestin in the uterus results in thickening of the cervical mucus, effectively blocking sperm from reaching an egg. Secondly, progestin prevents eggs from being released from the ovaries. In another example, the chemical may be non-hormonal, e.g. copper. In this example, the copper acts as a contraceptive by also thickening the cervical mucus, blocking the sperm from reaching an egg. Secondly, copper prevents a fertilised egg from being able to implant in the uterine lining.

For chemical disruption, the sensor 1 may either have a membrane coating that releases progestin or may alternatively have a copper wire wound on the body of the sensor 1.

For physical disruption, the sensor 1 is shaped to fit inside the uterus and contact at least part of the uterine lining in the uterus. This enables the sensor 1 to disrupt the growth of the uterine lining and therefore affect the implantation success of a fertilised egg to prevent pregnancy. As a uterus is typically pyriform in shape, the sensor 1 may be any suitable shape and size. For example, the shape of the sensor 1 may be T-shaped, serpentine shaped, oval shaped. Advantageously, the shape of the sensor 1 enables the sensor 1 to be anchored in the uterus.

As discussed earlier, the sensor 1 may use both chemical disruption and physical disruption means to act as a contraceptive device.

The smart IUD sensor 1 is capable of wirelessly receiving power from and wirelessly transmitting data to the wearable receiver 2 which is located outside the body of a user, and worn by the user. As a result, the smart IUD sensor 1 dispenses with the need for a battery and cables, and is of comparable size to standard IUDs for contraception. This is important, because for implantation in the uterus, a device must meet strict size limits. Some battery-based sensors have been found to be too large to be used in the uterus. Moreover, designs based on a battery typically have limitations due to the physical size of the battery and short lifetime before the battery is too depleted to continue operating. Furthermore, there are potential risks from the toxic material of a battery.

The receiver 2 serves as a medium between the IUD sensor device 1 and the external data processing device running suitable software (and thus operating as a data analyser). In particular, the receiver 2 delivers energy to the sensor device and collects real-time information. An antenna 4 of the receiver 2 can be embedded into clothing and wired to the receiver 2. The software module is developed for *in-vivo* data uploading simultaneously to smart terminals or PC servers for post data processing and analysis. The software module consists of a set of monitoring software running on a PC or smart terminal which is designed to be a friendly user interface for data processing and system configuration. The positioning of the smart IUD sensor 1 within the uterus is shown in Figure 1. In this example, the smart IUD sensor 1 has a generally elongate structure and is positioned substantially upright (vertical) within the uterus such that the longitudinal axis of the smart IUD sensor 1 is substantially vertical when the user is standing, although it will be appreciated that the vertical orientation may be achieved with other shapes such as oval..

In this three-module structured system, the effectiveness of the wireless energy transfer and data communication between the smart IUD sensor 1 and receiver 2 directly affect the usability of the intended system. An optimised design may not only result in better performance, smaller size, low power consumption and lower cost, but also improve end-user experience and clinical practise.

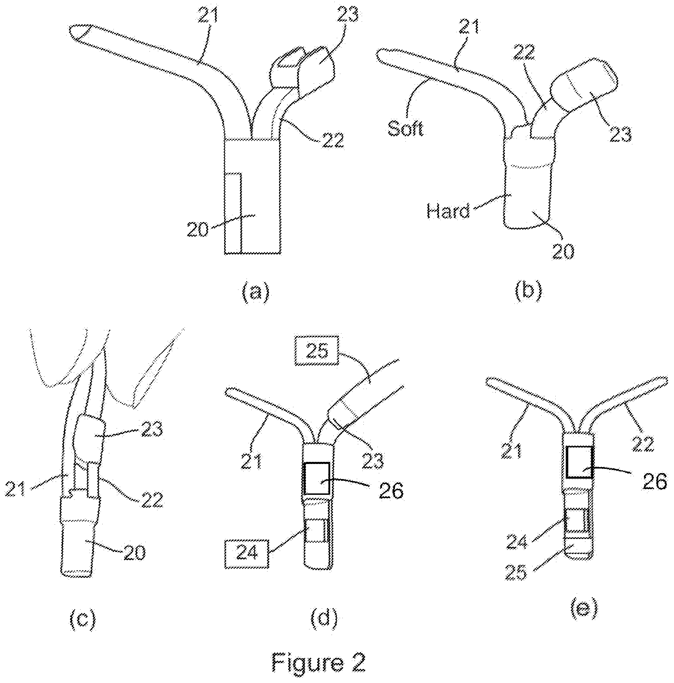

Referring to Figure 2, an example structure for the smart IUD sensor 1 is shown. In Figure 2(a), an upper portion of a T-type smart IUD sensor can be seen to comprise a middle connector 20, a first arm 21, and a second arm 22 having a connector 23. In Figure 2(b), it is explained that the middle connector 20 is formed of a different material with a different hardness than the first arm 21 and joint of the second arm 22. In particular, a relatively hard material is used for the main body and the connector 23, whereas a relatively soft material is used to form the first arm 21 and the joint on the second arm 22. From Figure 2(c) is it shown how the first and second arms 21, 22 are bent together during delivery into the uterus and removal to aid insertion/removal. Once inserted, the first and second arms 21, 22 help to retain the smart IUD sensor 1 in place within the uterus. While in the present embodiment two arms are used, it should be understood that in other embodiments a single arm could be used, or more than two arms could be used, or other shapes of the smart IUD sensor 1 may be used.

In Figure 2(d), it can be seen that a main circuit board 24 is connected to the middle connector 20 and an antenna 25 is fixed on the second arm 22 via the connector 23. The main circuit board 24 carries the sensors and the circuitry which will be described in detail below. In this arrangement, one of the arms serves both to help keep the smart IUD sensor 1 in place within the uterus, and also serves as the antenna for communicating with (and receiving power from) the receiver 2. The antenna 25 is generally horizontal within the uterus in the uterus, making it particularly effective for use with a receiver having an antenna embedded in upper garments. In alternative embodiments the antenna may not form part of an arm, but may be provided elsewhere. For example, the antenna may be in the middle connector portion 20 (such as shown in Figure 2(e)).

The antenna is wound on the inside of IUD sensor 1 arm 22 or the middle connector portion 20, the IUD sensor includes a coating or material portion 26 over the body of the sensor 1. The coating or material portion 26 contains a hormonal or non-hormonal contraceptive chemical, e.g. copper or progestin, and releases the contraceptive chemical, e.g. copper ions or progestin, over time. In this example, the antenna communicates with the receiver device 2 to provide sensor data, while the coating enables the sensor 1 to act as a contraceptive device. As described earlier, the hormonal or non-hormonal contraceptive chemical in the coating helps alter the intra-uterine environment to prevent pregnancy. The coating or material portion 26may be in the form of a copper winding. The coating or material portion 26 is in liquid contact with the physiological fluids in the uterine cavity and able to provide the contraceptive function.

Placing the antenna internally within the IUD is beneficial as the antenna is insulated from the physiological fluids in the uterine cavity. The physiological fluids in the uterine cavity have a high electrical conductivity and the failure to insulate the coils of the wireless antenna would render the antenna ineffective.

The internal antenna can be spatially separated from the contraceptive copper 26 providing the contraceptive function, or be fully or partially located under the coil or coating providing the contraceptive function.

Regardless of the positioning of the antenna in relation to the IUD sensor 1, the IUD sensor may be shaped and dimensioned to prevent pregnancy by physical disruption of the uterine lining (i.e. the endometrium).

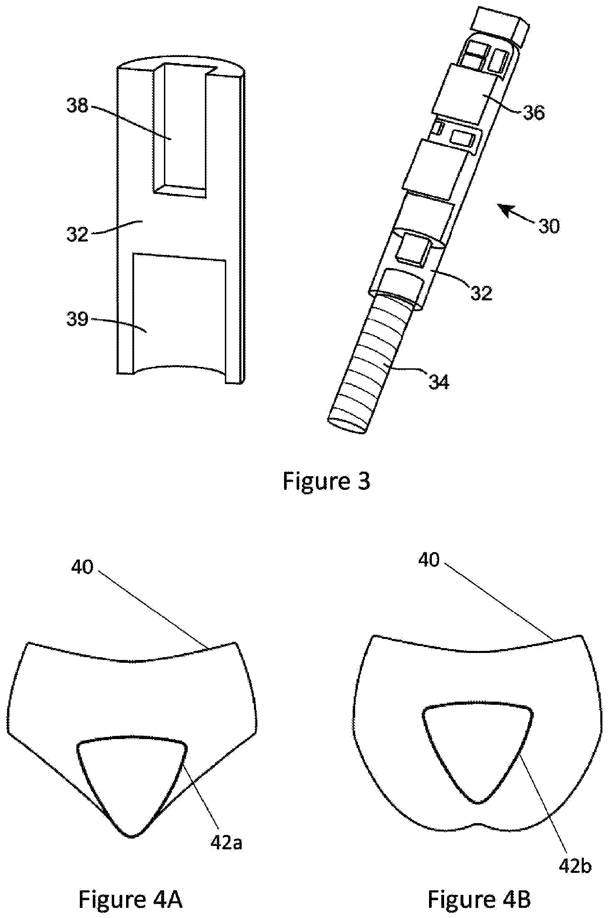

Referring to Figure 3, a simplified structure for the smart IUD sensor is shown. The left hand side of Figure 3 shows a cross section of a middle connector 32 and the right hand side of Figure 3 shows a full smart IUD sensor 30 including the internal connection of the middle connector 32. The middle connector 32 has two sockets, present here as a rectangular slot 38 for connection to a main circuit board 36 (with sensors and circuitry on board), and a circular slot 39 for connection to a tube antenna 34. As described earlier, the antenna 34 may be contained within tube 34, or it in the arms. The smart IUD sensor 30 may also further include a coating on a part of the device. For example, the coating may extend along only the middle connector 32, or may extend across the entire body of the device 30. The sensor device orientation of Figure 3 is vertical in the uterus, making it suitable for use with a receiver having a belt antenna or an antenna embedded in underwear (or a disposable sanitary towel). It will be appreciated that the sensor device of Figure 3 could be provided with one or more arms to assist with stabilising its position within the uterus if desired, or in any other suitable shape. Compared with other antenna types, a tube antenna can achieve a relatively small size and tight wind on a ferrite core because it does not require a coil frame. Considering fabrication complexity and the need for the sensor to be as small as possible, a tube antenna with a ferrite core is deemed as a particularly suitable antenna for the implantable sensor device. The antenna 34 may be a single axis antenna, formed as a coil antenna wound onto a core so that the wire is arranged circumferentially around only one axis of the core.

Figure 4A shows a receiver 2 in the form of a wearable garment comprising Helmholtz coils, in this case underwear 40. The front view is shown. The underwear 40 comprises a first Helmholtz coil 42a positioned on the front of the underwear 40. The first Helmholtz coil 42a is positioned so as to substantially conform with the wearer's body contours when worn, such as lying along the groove where the user's leg joins their pelvis. Figure 4B shows the back view of the underwear 40. The underwear 40 comprises a second Helmholtz coil 42b positioned on the back of the underwear 40.

Each Helmholtz coil is a generally planar wound wire that is protected from moisture ingress by a moisture sealing barrier, such as by sandwiching the wire between two moisture sealing layers. Typically, the Helmholtz coils comprise copper tracks provided on polyimide film.

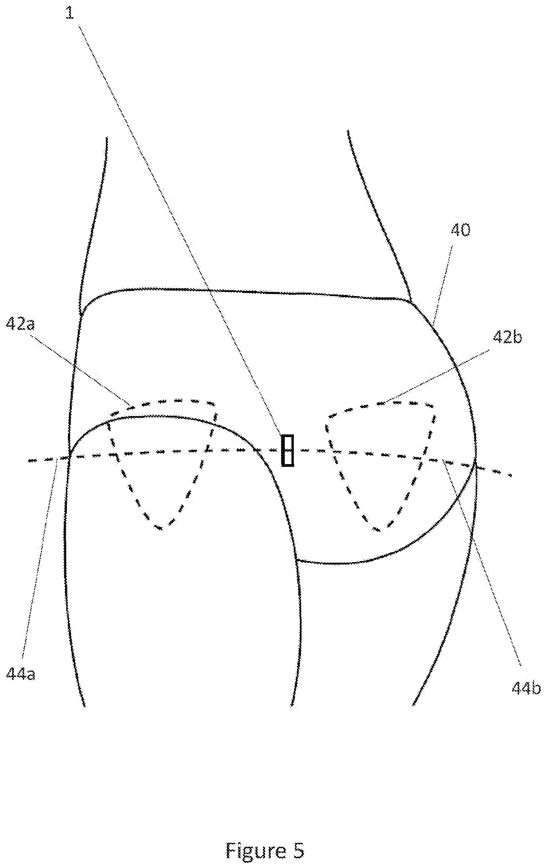

Figure 5 shows the underwear 40 being worn by a user. The user also has the IUD sensor 1 implanted in their uterus, as described above. The IUD sensor 1 has a single axis antenna. As described previously, the underwear 40 comprises first and second Helmholtz coils 42a, 42b located on the front and back of the underwear 40 respectively. The coils 42a, 42b are positioned such that the first Helmholtz coil 42a is positioned on a first side of the single axis antenna 34 and the second Helmholtz coil 42b is positioned on a second side of the single axis antenna 34. As the first Helmholtz coil 42a is positioned in a first side of the garment and the second Helmholtz coil 42b is positioned in a second side of the garment, it can be seen that when the garment is worn by the user, the first Helmholtz coil 42a is located on an opposite side of the user to the second Helmholtz coil 42b.

Each Helmholtz coil 42a, 42b defines a respective principal axis 44a, 44b passes through the centre of the coil and runs perpendicular to the plane of the coil. As can be seen, the principal axes 44a, 44b of the two Helmholtz coils 42a, 42b substantially intersect at the location of the IUD sensor 1. Therefore, when an electric current is applied to the Helmholtz coils, an extremely uniform magnetic field is generated between them, located in which is the IUD sensor 1. The IUD sensor 1 can therefore be wirelessly charged from this magnetic field using its single axis antenna.

The direction of the electric current in each of the coils can be applied in the same direction, clockwise or anticlockwise, through both front and back coils when viewed anteriorly to the body to generate a magnetic field that essentially aligns with a sensor single axis antenna that is located between the transverse plane of the body and close to, but not aligned to the coronal plane of the body. Alternatively, the direction of the electric current in each of the coils can be applied in a different direction through the front coil relative to the back coil to generate a magnetic field that essentially aligns with a sensor single axis antenna that is aligned to the coronal plane of the body. Thereby offering the ability to generate an appropriately-directioned magnetic field depending on the orientation of the single axis antenna. This orientation will likely depend on the orientation of the uterus in which the device is implanted, and it is typically not possible to alter this in vivo orientation. It is therefore important to be able to control the orientation of the applied magnetic field, in the manner described.

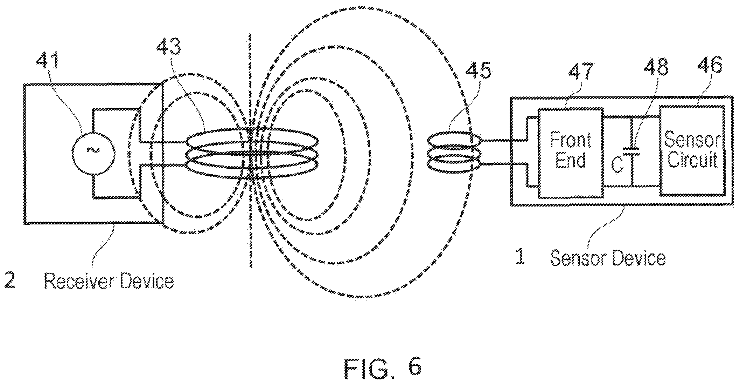

Referring to Figure 6, a setup which illustrates the manner in which the receiver 2 and IUD sensor 1 can be expected to communicate and transfer power is shown. In Figure 4, electrical power, generated by an AC source 41, is transferred from the receiver 2 to an inductively coupled IUD sensor 1 using the energy of the alternating magnetic field generated by the receiver 2. Power transfer in Figure 4 is achieved by the principle of magnetic induction, where both wireless power transfer to the IUD sensor 1 and communication between the two devices are achieved using the same nearfield magnetic induction link.

The receiver 2 drives a primary coil 43 at low frequency to generate a magnetic field (in this case at a frequency of order 100kHz). The IUD sensor 1 has a secondary coil 45 in the vicinity of the primary (receiver) coil 43, which is inductively coupled to the receiver coil 43 (via the magnetic field H). This results in an induced voltage across the IUD sensor coil 45 which is then converted to energy by a front end 47 to power an integrated circuit 46 of the IUD sensor 1. More particularly, the front end 47 uses the energy received at the coil 45 to charge a capacitor 48, which can subsequently be used to power the sensor circuit 46. To allow high power transfer efficiency over distance, both the receiver 2 and IUD sensor 1 are preferably designed to operate their antenna circuits at the same resonant frequency.

The communication between the receiver 2 and the IUD sensor 1 is achieved using the same pair of coils 43, 45 as for powering the IUD sensor 1. When the receiver or the IUD sensor 1 manipulate an applied voltage to change the amplitude, frequency or phase of the voltage on one side, the other side will detect a similar change via the inductive link between the two devices. The message is encoded in this change, and how fast the message can be sent, or the data rate, is dependent on the bandwidth of this inductive link. Any form of modulation (amplitude, frequency or phase) will generate sidebands in the frequency domain, which makes the modulated signal difficult or impossible to detect if the frequency spectrum of the modulated signal does not fall wholly or substantially within the bandwidth of the inductive link. In the present embodiment, messages transmitted, via the inductive link, from the receiver 2 to the IUD sensor 1 may be command messages to trigger the IUD sensor 1 to take sensor readings (for example), while the messages transmitted, via the inductive link, from the IUD sensor 1 to the receiver 2 may be sensor data generated by a pH sensor, temperature sensor or dissolved oxygen sensor (for example), or status messages indicating the current status (e.g. power level) of the IUD sensor 1.

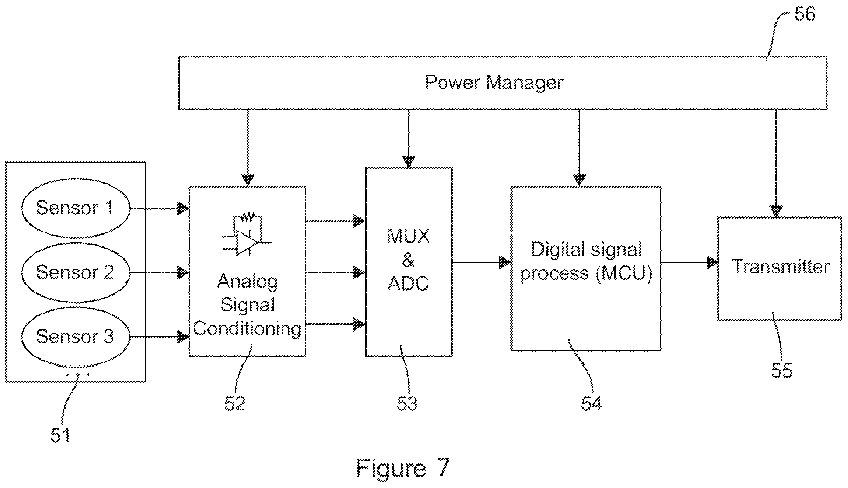

Referring to Figure 7, a block diagram of a suitable microsystem structure for the IUD sensor 1 is shown. To achieve data capture, signal processing and wireless communication, the microsystem includes integrated sensors or a multi-sensor array 51, an analogue signal conditioning circuit 52, an analogue to digital converter (ADC) 53 optionally including a multiplexer (MUX), a digital signal processor 54 or micro-programmed control unit (MCU), a wireless transmitter 55 and a power manager 56. In operation, the sensors 51 convert physical parameters into electronic signals. Sensors can be fabricated on tiny silicon chips based on micro-fabrication technologies which are suitable for microsystems. The conditioning circuit 52 is used to improve the quality of the analogue signals from the sensors 51. Generally, high end conditioning performance requires more complex circuits. A simpler conditioning circuit results in limited performance that requires further data processing after analogue-digital conversion. Therefore, a balance between signal conditioning performance and circuit complexity should be considered for the system implementation. A multiplexer (MUX) may be employed for circuit hardware sharing, which has the advantage of reducing device size and power consumption. The processer 54 undertakes logic control and digital signal processing. A micro-programmed control unit (MCU) is a widely-used component for flexible functionality and good scalability. At the same time, compared with a powerful processor, an MCU enables power consumption to be reduced. The wireless transmitter 55 and power manager 56 are provided for data transmission and power control respectively. Integration of these different units can facilitate device miniaturisation.

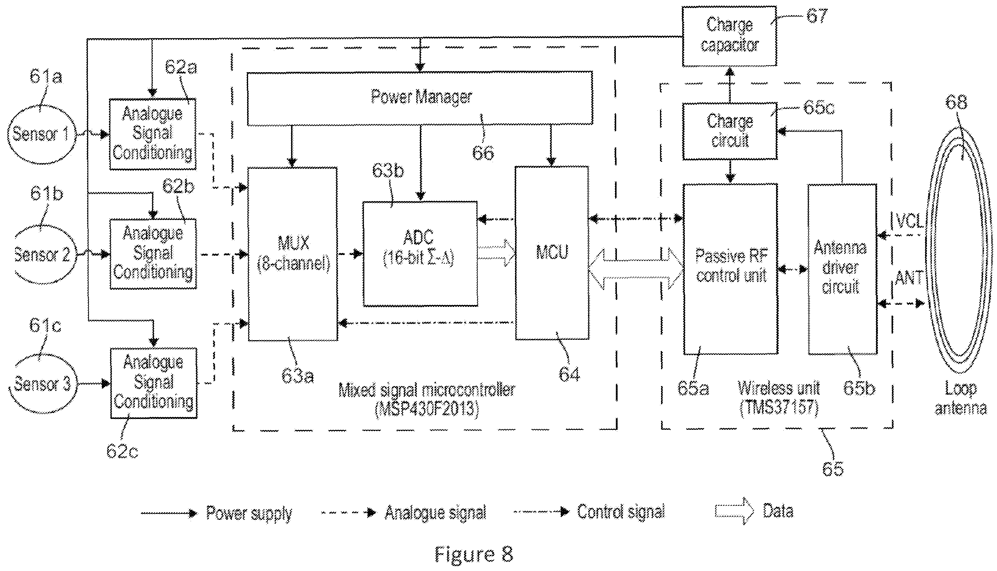

Referring to Figure 8, the main structure of the IUD sensor 1 is shown in greater detail, including sensors 61a, 61b, 61c (hormones, proteins, glucose, lactate, ions,, temperature, DOC and pH in this case), respective analogue signal conditioning units 62a, 62b, 62c (one for conditioning the output of each of the sensors 61a, 61b, 61c), a multiplexer 63a (to allow an array of sensors to be interfaced with the ADC), analogue to digital converter 63b and MCU 64, and a transmitter 65. Two-way communication with the MCU from outside the body is also possible, for example to wake it from a power saving sleep-state. Furthermore, the antenna can be used for wireless power transfer through inductive coupling. The transmitter 65 here comprises a passive RF control unit 65a for communicating with the MCU 64, and controlling an antenna driver circuit 65b and a charging circuit 65c. The antenna driver circuit is capable of bidirectional data communication with the external receiver (Antenna, ANT) via an antenna (in this case a loop antenna) 68, as well as receiving power (charging voltage, VCL) via the antenna 68. It will therefore be understood that the system utilises wireless power transfer through inductive coupling, using a low frequency RFID signal to transmit data and receive power through the abdominal region of a user. The charge circuit 65c is able to charge a capacitor 67 and also deliver power to the control unit 65a using the electrical power received via the antenna 68 and driver circuit 65b. All power is managed through the power management block 66. The flow of power around the circuitry of Figure 8 is indicated by solid directional arrows. Analogue data signal flow is indicted by a first type of dashed directional arrow. Control signal flow is indicated by a second type of dashed directional arrow. Data flow is indicated by solid block arrows. It can be seen that the charge capacitor 67 delivers electrical power to the power manager 66 (which in turn manages power delivery to the multiplexer 63a, the ADC 63b and the MCU 64), as well as to the signal conditioning units 62a, 62b, 62c. The capacitor 67 is preferably a ceramic capacitor. This type of capacitor is particularly suitable for the present purposes for safety reasons, for example because it does not utilise toxic materials and has little or no risk of leakage.

The receiver device has a primary coil for wirelessly communicating with the IUD sensor, and a quality factor of the primary coil is controllable to be higher when the receiver device is wirelessly charging the IUD sensor than when the receiver device is receiving sensor data from the IUD sensor.

As a result of the quality factor being controllable, the same coil can be used both for efficient power transfer (wireless charging) by using the coil in a (relatively) high quality factor mode, and for reliable data communications by using the coil in a (relatively) low quality factor mode.

The quality factor may be controllable by modifying an electrical resistance associated with the primary coil. For example, a damping resistor could be switched in and out of series with the primary coil.

The quality factor of the primary coil may be selectable between a plurality of different levels. In a simple case, this could be two levels - a high level for wireless charging and a low level for receiving and/or transmitting data. In a more complicated case, this could be three levels, with an intermediate quality factor being used, for example if a different quality factor would be optimal for transmitting data compared with receiving data. For example, different data rates may be used for transmitting and receiving. If a faster data rate is used for receiving, then receiving will require a higher bandwidth and thus a lower quality factor. Another reason for providing multiple different quality factors is to provide robustness to the communication link when the user is close to a metallic environment. For example, if the user is sitting in a car or sitting in a metal chair, then if the system is operating at a high quality factor, the presence of metal may detune the system, causing one or both of power transfer and communication to fail. However, a system with a lower quality factor will waste more power, which is generally undesirable, and may be unacceptable for a receiver device, where power consumption needs to be relatively low. In order to alleviate this problem, the system may be configured to adaptively change the quality factor to accommodate the external environment while maintaining a reasonable battery life.

The receiver device 2 may include user interface comprising an LCD display and a keyboard is able to display received sensor data and system information provided by the microcontroller, and to offer a facility for a user to operate the device, again via the microcontroller. A Bluetooth module may provide high-level communication with servers or smart terminals, where data analysis can be performed.

The IUD sensor may be operable for single sampling or continuous sampling. The IUD may be charged wirelessly from the receiver device in multiple steps for each sampling event, or the IUD sensor may alternatively be charged on demand so that IUD is wirelessly charged from the receiver device whenever the stored power in the IUD falls below a threshold.

The low power state may be a "sleep" state in which the device is substantially powered down to conserve power, but is capable of being woken up to operate. In contrast, the idle state may be an operational state in which the device is merely awaiting an instruction. The device may be quicker to react when in the idle state than in the lower power (sleep) state.

As discussed above, electromagnetic induction wireless transmission technology is used for near field energy transfer through the use of two coupled coils, primary and secondary coils, provided at the receiver device and implantable IUD sensor respectively. The electric current flowing through the primary coil creates a magnetic field that acts on the secondary coil producing an induced current within it. Tight coupling is needed for high energy harvest efficiency and long working distance. Increasing the distance between the coils results in the magnetic field extending beyond the secondary coil receiving area and leads to a loss of transmitted energy. Within the intended application, an implantable IUD sensor requires small size, low power consumption and a relatively short working distance of around 10cms. This enables long term monitoring parameters in the uterus. When the IUD sensor 1 uses a hormonal coating, ideally, the sensor is replaced after the hormonal coating has depleted. Typically, this can be between 3-7 years after implantation.

Energy loss due to tissue absorption of the wireless signals (the electromagnetic energy is transformed to other forms of energy by matter within the medium of tissues, for example, to heat) is dependent on the signal frequency. Signals at lower frequencies have better propagation characteristics and result in less tissue absorption. Therefore, wireless energy transfer based on electromagnetic induction at low frequency is employed for the implantable IUD sensor. The circuit for wireless energy transfer can also serve as the wireless data communication as a low frequency RFID link, reducing the need for additional circuits or board space for data communication. In the intended application, the *in-vivo* information and system configuration do not require high data rate transmission and the LF RFID link can provide sufficient data bandwidth to meet the demand. The data communication range is usually further than the energy transfer distance meaning it is not the bottleneck of the effective working distance.

Monitoring changes of the biophysical parameters in the uterus enables the IUD sensor 1 to be used for diagnosing conditions.

While the various techniques, and the implantable IUD sensor and external receiver have been explained in the context of intra-uterine monitoring, it will be understood that these techniques and structures could be applied to other body-cavity monitoring, such as within a vagina, bladder or digestive tract of a human or animal body.

## Claims

1. An intra-uterine system, comprising:
an intra-uterine device (1), shaped and dimensioned for implantation in a uterus (5); and
an external receiver device (2),
wherein the intra-uterine device is for measuring conditions within the uterus to generate sensor data
wherein the receiver device is for wirelessly receiving the sensor data generated by the intra-uterine device, **characterised in that** the intra-uterine device is further configured for acting as a contraceptive device to prevent pregnancy,
wherein the intra-uterine device has an antenna (25) arranged internally within the intra-uterine device and
wherein the intra-uterine device has a contraceptive copper portion (26) provided externally on the intra-uterine device.

2. An intra-uterine system according to claim 1, wherein the receiver device (2) is operable to wirelessly charge a implantable sensor device (1).

3. An intra-uterine system according to any preceding claim, wherein the intra-uterine device (1) is shaped so as to anchor in the uterus (5).

4. An intra-uterine system according to any preceding claim, wherein the intra-uterine device (1) is shaped to contact at least part of the uterine lining in the uterus (5);
preferably wherein the at least part of the intra-uterine device which contacts the uterine lining disrupts the growth of the uterine lining so as to affect implantation of a fertilised egg to prevent pregnancy.

5. An intra-uterine system according to any preceding claim, wherein the intra-uterine device (1) releases a chemical into the uterus (5) to disrupt the intra-uterine environment;
preferably wherein the chemical is provided in or as a coating of a part of the intra-uterine device, or in or as a part of the intra-uterine device; and
preferably wherein the chemical is hormonal or nonhormonal, preferably one or more of copper, progestin or estrogen.

6. An intra-uterine system according to any preceding claim, wherein the receiver device (2) comprises an antenna (4), transceiver circuitry and a power source, and the intra-uterine device comprises an antenna, a charging circuit and a controller, wherein the receiver device is operable to transmit electrical power from the antenna of the receiver device to the antenna (25) of the intra-uterine device (1) via electromagnetic coupling, the electrical power being used by the charging circuit to store electrical power for operating the sensors of the intra-uterine device and for transmitting sensor data to the receiver device.

7. An intra-uterine system according to claim 6, wherein the intra-uterine device comprises a capacitor (48) or a rechargeable battery, and the charging circuit stores the electrical power by charging the capacitor or the rechargeable battery; and/or
wherein the power source is a rechargeable battery.

8. An intra-uterine system according to any preceding claim, wherein the receiver device (2) has a controller, and a primary coil (43) for wirelessly communicating with the intra-uterine device, the receiver device being operable to wirelessly charge the intra-uterine device (1) via inductive coupling between the primary and secondary coils (45); wherein:
a quality factor of the primary coil is controllable;
the controller is operable to control the quality factor of the primary coil to be higher when the receiver device is wirelessly charging the intra-uterine device than when the receiver device is receiving sensor data from the intra-uterine device; and
the receiver device drives the primary coil to operate at a frequency of order 100kHz.

9. An intra-uterine system according to any preceding claim, wherein the intra-uterine device (1) comprises a single axis antenna (25), and wherein the receiver device (2) comprises Helmholtz coils (42a), wherein the Helmholtz coils are arranged to communicate with the single axis antenna so as to wirelessly charge the intra-uterine device and receive the sensor data;
preferably wherein the single axis antenna is configured to operate at a frequency in the range 119kHz to 140kHz, preferably 130kHz to 140kHz, preferably 139.6kHz.

10. An intra-uterine system according to any preceding claim, wherein the intra-uterine device (1) comprises one or more of a temperature sensor, a pH sensor and a dissolved oxygen sensor; and/or
wherein the intra-uterine device comprises one or both of an electrical conductivity sensor and a pressure sensor.

11. An intra-uterine system according to any preceding claim, wherein the intra-uterine device comprises a body (23) and one or more arms (21, 22), the arms projecting laterally from the body to secure the sensor within the uterus (5);
preferably wherein the intra-uterine device comprises a pair of arms positioned at or proximate one end of the body and extending generally away from each other.

12. An intra-uterine system according to any preceding claim, wherein the receiver device (2) is a wearable receiver device; and/or
wherein the receiver device is provided in a garment;
preferably wherein the garment is underwear worn adjacent the uterus.

13. An intra-uterine system according to any one of claims 1 to 11, wherein the receiver device (2) is a mat or blanket.

14. An intra-uterine system according to any preceding claim, wherein the receiver device (2) comprises a transmitter (55) for wirelessly transmitting received sensor data to an external device;
preferably wherein the external device is a portable electronic device (3b) or a computer (3a).

15. An intra-uterine system according to any preceding claim, wherein the contraceptive copper portion is spatially separated from the antenna (25) such that the contraceptive copper portion does not impede the antenna operation.

## Patentansprüche

1. Intrauterines System, umfassend:
eine intrauterine Vorrichtung (1), die zum Implantieren in einen Uterus (5) geformt und bemessen ist; und
eine äußere Empfangsvorrichtung (2),
wobei die intrauterine Vorrichtung zum Messen von Bedingungen innerhalb des Uterus zur Erzeugung von Sensordaten dient
wobei die Empfangsvorrichtung zum drahtlosen Empfangen der von der intrauterinen Vorrichtung erzeugten Sensordaten dient, **dadurch gekennzeichnet, dass** die intrauterine Vorrichtung weiter dafür ausgebildet ist, als empfängnisverhütende Vorrichtung zu wirken, um eine Schwangerschaft zu verhindern,
wobei die intrauterine Vorrichtung eine Antenne (25) aufweist, die intern innerhalb der intrauterinen Vorrichtung angeordnet ist und
wobei die intrauterine Vorrichtung einen empfängnisverhütenden Kupferabschnitt (26) aufweist, der extern an der intrauterinen Vorrichtung bereitgestellt ist.

2. Intrauterines System nach Anspruch 1, wobei die Empfangsvorrichtung (2) betriebsfähig ist, eine implantierbare Sensorvorrichtung (1) drahtlos zu laden.

3. Intrauterines System nach einem vorstehenden Anspruch, wobei die intrauterine Vorrichtung (1) so geformt ist, dass sie im Uterus (5) verankert wird.

4. Intrauterines System nach einem vorstehenden Anspruch, wobei die intrauterine Vorrichtung (1) so geformt ist, dass sie mit zumindest einem Teil der Gebärmutterschleimhaut im Uterus (5) in Kontakt steht;
bevorzugt, wobei der zumindest eine Teil der intrauterinen Vorrichtung, der mit der Gebärmutterschleimhaut in Kontakt steht, das Wachstum der Gebärmutterschleimhaut stört, sodass die Implantation einer befruchteten Eizelle beeinflusst wird, um eine Schwangerschaft zu verhindern.

5. Intrauterines System nach einem vorstehenden Anspruch, wobei die intrauterine Vorrichtung (1) eine chemische Substanz in den Uterus (5) freisetzt, um die intrauterine Umgebung zu stören;
bevorzugt, wobei die chemische Substanz in oder als eine Beschichtung eines Teils der intrauterinen Vorrichtung, oder in oder als ein Teil der intrauterinen Vorrichtung bereitgestellt ist; und
bevorzugt, wobei die chemische Substanz hormonell oder nichthormonell ist, bevorzugt eines oder mehrere von Kupfer, Gestagen oder Östrogen.

6. Intrauterines System nach einem vorstehenden Anspruch, wobei die Empfangsvorrichtung (2) eine Antenne (4), eine Sende-/Empfangsschaltung und eine Energiequelle umfasst, und die intrauterine Vorrichtung eine Antenne eine Ladeschaltung und eine Steuereinheit umfasst, wobei die Empfangsvorrichtung betriebsfähig ist, elektrische Leistung von der Antenne der Empfangsvorrichtung zu der Antenne (25) der intrauterinen Vorrichtung (1) über elektromagnetische Kopplung zu übertragen, wobei die elektrische Leistung von der Ladeschaltung verwendet wird, um elektrische Leistung zum Betreiben der Sensoren der intrauterinen Vorrichtung und zum Übertragen von Sensordaten zu der Empfangsvorrichtung zu speichern.

7. Intrauterines System nach Anspruch 6, wobei die intrauterine Vorrichtung einen Kondensator (48) oder eine wiederaufladbare Batterie umfasst und die Ladeschaltung die elektrische Leistung durch Laden des Kondensators oder der wiederaufladbaren Batterie speichert; und/oder
wobei die Energiequelle eine wiederaufladbare Batterie ist.

8. Intrauterines System nach einem vorstehenden Anspruch, wobei die Empfangsvorrichtung (2) eine Steuereinheit und eine Primärspule (43) zum drahtlosen Kommunizieren mit der intrauterinen Vorrichtung aufweist, wobei die Empfangsvorrichtung betriebsfähig ist, die intrauterine Vorrichtung (1) drahtlos über induktive Kopplung zwischen der Primär- und Sekundärspule (45) zu laden; wobei:
ein Gütefaktor der Primärspule regelbar ist;
die Steuereinheit betriebsfähig ist, den Gütefaktor der Primärspule derart zu regeln, dass er höher ist, wenn die Empfangsvorrichtung die intrauterine Vorrichtung drahtlos lädt, als wenn die Empfangsvorrichtung Sensordaten von der intrauterinen Vorrichtung empfängt; und
die Empfangsvorrichtung die Primärspule antreibt, um bei einer Frequenz in der Größenordnung von 100 kHz zu arbeiten.

9. Intrauterines System nach einem vorstehenden Anspruch, wobei die intrauterine Vorrichtung (1) eine einachsige Antenne (25) umfasst, und wobei die Empfangsvorrichtung (2) Helmholtz-Spulen (42a) umfasst, wobei die Helmholtz-Spulen dazu angeordnet sind, mit der einachsigen Antenne zu kommunizieren, um die intrauterine Vorrichtung drahtlos zu laden und die Sensordaten zu empfangen;
bevorzugt, wobei die einachsige Antenne ausgebildet ist, bei einer Frequenz im Bereich 119 kHz bis 140 kHz, bevorzugt 130 kHz bis 140 kHz, bevorzugt 139,6 kHz zu arbeiten.

10. Intrauterines System nach einem vorstehenden Anspruch, wobei die intrauterine Vorrichtung (1) einen oder mehrere von einem Temperatursensor, einem pH-Sensor und einem Sensor für gelösten Sauerstoff umfasst; und/oder
wobei die intrauterine Vorrichtung einen oder beide von einem Sensor für elektrische Leitfähigkeit und einem Drucksensor umfasst.

11. Intrauterines System nach einem vorstehenden Anspruch, wobei die intrauterine Vorrichtung einen Körper (23) und einen oder mehrere Arme (21, 22) umfasst, wobei die Arme seitlich von dem Körper abragen, um den Sensor innerhalb des Uterus (5) zu sichern;
bevorzugt, wobei die intrauterine Vorrichtung ein Paar von Armen umfasst, die an oder nahe einem Ende des Körpers positioniert sind und sich im Allgemeinen voneinander weg erstrecken.

12. Intrauterines System nach einem vorstehenden Anspruch, wobei die Empfangsvorrichtung (2) eine tragbare Empfangsvorrichtung ist; und/oder
wobei die Empfangsvorrichtung in einem Kleidungsstück bereitgestellt ist;
bevorzugt, wobei das Kleidungsstück Unterwäsche ist, die angrenzend an den Uterus getragen wird.

13. Intrauterines System nach einem der Ansprüche 1 bis 11, wobei die Empfangsvorrichtung (2) eine Matte oder Decke ist.

14. Intrauterines System nach einem vorstehenden Anspruch, wobei die Empfangsvorrichtung (2) eine Übertragungsvorrichtung (55) zum drahtlosen Übertragen der empfangenen Sensordaten an eine externe Vorrichtung umfasst;
bevorzugt, wobei die externe Vorrichtung eine tragbare elektronische Vorrichtung (3b) oder ein Computer (3a) ist.

15. Intrauterines System nach einem vorstehenden Anspruch, wobei der empfängnisverhütende Kupferabschnitt räumlich von der Antenne (25) getrennt ist, sodass der empfängnisverhütende Kupferabschnitt den Betrieb der Antenne nicht beeinträchtigt.

## Revendications

1. Système intra-utérin, comprenant :
un dispositif intra-utérin (1), formé et dimensionné pour une implantation dans un utérus (5) ; et
un dispositif récepteur externe (2),
dans lequel le dispositif intra-utérin est destiné à mesurer des conditions à l'intérieur de l'utérus pour générer des données de capteur
dans lequel le dispositif récepteur est destiné à recevoir sans fil les données de capteur générées par le dispositif intra-utérin, **caractérisé en ce que** le dispositif intra-utérin est en outre configuré pour agir comme dispositif contraceptif pour empêcher une grossesse,
dans lequel le dispositif intra-utérin présente une antenne (25) agencée de façon interne à l'intérieur du dispositif intra-utérin et
dans lequel le dispositif intra-utérin présente une partie en cuivre contraceptive (26) disposée de façon externe sur le dispositif intra-utérin.

2. Système intra-utérin selon la revendication 1, dans lequel le dispositif récepteur (2) est utilisable pour recharger sans fil un dispositif de capteur implantable (1).

3. Système intra-utérin selon une quelconque revendication précédente, dans lequel le dispositif intra-utérin (1) est formé de manière à s'ancrer dans l'utérus (5).

4. Système intra-utérin selon une quelconque revendication précédente, dans lequel le dispositif intra-utérin (1) est formé pour entrer en contact avec au moins une partie de la muqueuse utérine dans l'utérus (5) ;
de préférence dans lequel au moins une partie du dispositif intra-utérin qui entre en contact avec la muqueuse utérine perturbe la croissance de la muqueuse utérine de manière à affecter l'implantation d'un ovule fécondé afin d'empêcher une grossesse.

5. Système intra-utérin selon une quelconque revendication précédente, dans lequel le dispositif intra-utérin (1) libère un agent chimique dans l'utérus (5) de manière à perturber l'environnement intra-utérin ;
de préférence dans lequel l'agent chimique est disposé dans une partie du dispositif intra-utérin ou en tant que revêtement de celui-ci, ou dans le dispositif intra-utérin ou en tant que partie de celui-ci ; et
de préférence dans lequel l'agent chimique est hormonal ou non hormonal, de préférence l'un ou plusieurs parmi le cuivre, un progestatif ou un estrogène.

6. Système intra-utérin selon une quelconque revendication précédente, dans lequel le dispositif récepteur (2) comprend une antenne (4), des circuits émetteur-récepteur et une source d'alimentation, et le dispositif intra-utérin comprend une antenne, un circuit de charge et un dispositif de commande, dans lequel le dispositif récepteur est utilisable pour transmettre une puissance électrique depuis l'antenne du dispositif récepteur vers l'antenne (25) du dispositif intra-utérin (1) par le biais d'un couplage électromagnétique, la puissance électrique étant utilisée par le circuit de charge pour stocker de la puissance électrique pour faire fonctionner les capteurs du dispositif intra-utérin et pour transmettre des données de capteur au dispositif récepteur.

7. Système intra-utérin selon la revendication 6, dans lequel le dispositif intra-utérin comprend un condensateur (48) ou une batterie rechargeable, et le circuit de charge stocke la puissance électrique par le chargement du condensateur ou de la batterie rechargeable ; et/ou
dans lequel la source d'alimentation est une batterie rechargeable.

8. Système intra-utérin selon une quelconque revendication précédente, dans lequel le dispositif récepteur (2) présente un dispositif de commande, et une bobine primaire (43) pour communiquer sans fil avec le dispositif intra-utérin, le dispositif récepteur étant utilisable pour recharger sans fil le dispositif intra-utérin (1) par le biais d'un couplage inductif entre les bobines primaire et secondaire (45) ; dans lequel :
un facteur de qualité de la bobine primaire est commandable ;
le dispositif de commande est utilisable pour commander le facteur de qualité de la bobine primaire pour qu'il soit plus élevé lorsque le dispositif récepteur recharge sans fil le dispositif intra-utérin que lorsque le dispositif récepteur reçoit des données de capteur en provenance du dispositif intra-utérin ; et
le dispositif récepteur entraîne la bobine primaire pour fonctionner à une fréquence de l'ordre de 100 kHz.

9. Système intra-utérin selon une quelconque revendication précédente, dans lequel le dispositif intra-utérin (1) comprend une antenne à axe unique (25), et dans lequel le dispositif récepteur (2) comprend des bobines de Helmholtz (42a), dans lequel les bobines de Helmholtz sont conçues pour communiquer avec l'antenne à axe unique de manière à recharger sans fil le dispositif intra-utérin et à recevoir les données de capteur ;
de préférence dans lequel l'antenne à axe unique est configurée pour fonctionner à une fréquence dans la plage de 119 kHz à 140 kHz, de préférence de 130 kHz à 140 kHz, de préférence 139,6 kHz.

10. Système intra-utérin selon une quelconque revendication précédente, dans lequel le dispositif intra-utérin (1) comprend un ou plusieurs parmi un capteur de température, un capteur de pH et un capteur d'oxygène dissous ; et/ou
dans lequel le dispositif intra-utérin comprend l'un ou les deux parmi un capteur de conductivité électrique et un capteur de pression.

11. Système intra-utérin selon une quelconque revendication précédente, dans lequel le dispositif intra-utérin comprend un corps (23) et un ou plusieurs bras (21, 22), les bras s'étendant latéralement depuis le corps afin de maintenir le capteur en place à l'intérieur de l'utérus (5) ;
de préférence dans lequel le dispositif intra-utérin comprend une paire de bras positionnés au niveau ou à proximité d'une extrémité du corps et s'étendant globalement l'un à l'opposé de l'autre.

12. Système intra-utérin selon une quelconque revendication précédente, dans lequel le dispositif récepteur (2) est un dispositif récepteur portable ; et/ou
dans lequel le dispositif récepteur est disposé dans un vêtement ;
de préférence dans lequel le vêtement est un sous-vêtement porté à proximité de l'utérus.

13. Système intra-utérin selon l'une quelconque des revendications 1 à 11, dans lequel le dispositif récepteur (2) est un tapis ou une couverture.

14. Système intra-utérin selon une quelconque revendication précédente, dans lequel le dispositif récepteur (2) comprend un émetteur (55) pour transmettre sans fil des données de capteur reçues à un dispositif externe ;
de préférence dans lequel le dispositif externe est un dispositif électronique portable (3b) ou un ordinateur (3a).

15. Système intra-utérin selon une quelconque revendication précédente, dans lequel la partie en cuivre contraceptive est séparée spatialement de l'antenne (25) de sorte que la partie en cuivre contraceptive n'entrave pas le fonctionnement de l'antenne.
